# EUROPEAN PATENT APPLICATION

(11) **EP 4 393 443 A1**
(43) Date of publication of application: **03.07.2024**
(21) Application number: 22897306.1
(22) Date of filing: 09.09.2022
(51) Int. Cl.: A61B 34/30

(54) **INSTRUMENT DRIVER FOR SURGICAL ROBOT AND SURGICAL ROBOT**

(30) Priority: 29.11.2021 CN 202111435643
(71) Applicant: Cornerstone Technology (Shenzhen) Limited, Shenzhen, Guandong 518066 (CN)
(72) Inventor: REN, Lixue, Shenzhen, Guangdong 518066 (CN); LU, Yueqiang, Shenzhen, Guangdong 518066 (CN)
(74) Representative: Balder IP Law, S.L.
(86) International application number: PCT/CN2022/118017
(87) International publication number: WO 2023/093218

(57) **Abstract**

An instrument drive for a surgical robot and the surgical robot are provided. The instrument drive includes a motor, a reducer, a reducer output stage, and a driving disc assembly. The motor is configured to drive a gear of the reducer to rotate, and the reducer is configured to drive the driving disc assembly to rotate. The instrument drive further includes an output stage magnetic ring and an output stage encoder. The output stage magnetic ring is coaxially arranged with the reducer output stage, and is configured to rotate synchronously with the reducer output stage. The output stage encoder is fixedly arranged in a radial direction of the reducer output stage and configured to detect the rotation of the output stage magnetic ring.

## Description

### CROSS REFERENCE TO RELATED APPLICATIONS

This application claims priority to Chinese Patent Application No. CN202111435643.5, entitled "Instrument Drive for Surgical Robot and Surgical Robot," filed on November 29, 2021, which is hereby incorporated by reference in its entirety.

### TECHNICAL FIELD

The various embodiments described in this document relate in general to the technical field of surgical robots, and more specifically to an instrument drive for a surgical robot and the surgical robot.

### BACKGROUND

Medical surgical robots are widely used in various surgeries due to having the advantages of accurate positioning, stable operation, good dexterity, large working range, radiation and infection protection, etc. The use of the medical surgical robots is helpful to improve the precision of surgeons' operations, solve the tremors, fatigue, and musculoneurological feedback of the surgeons' hands, and enable the surgeons to operate in the most comfortable state, which is of great value to improve the success rate of surgery and alleviate the pain of patients. In recent years, the research on the medical surgical robot has become a new field of medical device application.

The surgical robot generally includes driving devices for surgical instruments arranged at ends of one or more robotic arms of the surgical robot. The driving devices need to be adapted to various surgical instruments which may be replaced during a surgery and in different types of surgeries to complete various surgical actions in the surgeries.

### SUMMARY

A series of simplified concepts have been introduced in this section, which will be further elaborated in the detailed description. The section of the disclosure does not mean attempting to limit key features and essential technical features of the claimed technical solution, nor does it mean attempting to determine the scope of protection of the claimed technical solution.

According to a first aspect of the disclosure, embodiments of the disclosure provide an instrument drive for a surgical robot, the instrument drive including a plurality of motors, a plurality of reducers, a plurality of reducer output stages, and a plurality of driving disc assemblies. Each respective motor of the plurality of motors is configured to drive a respective reducer to rotate a respective driving disc assembly via a respective reducer output stage. The instrument drive further includes: a plurality of output stage magnetic rings. A respective output stage magnetic ring of the plurality of output stage magnetic rings is coaxially disposed with the respective reducer output stage and configured to rotate synchronously with the respective reducer output stage; and a plurality of output stage encoders. A respective output stage encoder of the plurality of output stage encoders is fixedly disposed in a radial direction of the respective reducer output stage and configured to detect rotation of the respective output stage magnetic ring.

In the instrument drive for the surgical robot of the disclosure, the output stage magnetic ring is coaxial with the reducer output stage, and the output stage encoder is disposed in the radial direction of the reducer output stage, so that the detection of the rotational position of the driving disc can be realized at an axial height of less than 4.5mm and within a space of 7.5mm from the central axis of the reducer in the radial direction of the reducer. In this way, the internal structure of the instrument driver can be simplified, and the internal space of the instrument drive can be greatly saved, so that the external dimension of the instrument drive is smaller and the structure of the surgical robot is better. In addition, in the disclosure, errors such as clearance or deformation caused by complex structure can be eliminated, the rotation angle of the output stage can be directly measured, measurement of the rotation angle of the output stage is more accurate, and control accuracy for end instruments is higher.

In some embodiments, the instrument drive further includes an output sensing plate disposed between an output stage bearing of the respective reducer and the respective driving disc assembly, and the output sensing plate defines a plurality of through holes, where a respective through hole allows the respective reducer output stage to pass through. The plurality of output stage encoders are mounted to the output sensing plate.

In the instrument drive for the surgical robot of the disclosure, the respective output stage encoder is mounted between the respective driving disc assembly and the output stage bearing of the respective reducer.

In some embodiments, the respective output stage magnetic ring is mounted to the respective driving disc assembly and the respective output stage encoder is mounted to a side of the output sensing plate facing the respective driving disc assembly.

In the instrument drive for the surgical robot of the disclosure, the output stage encoder can be located below the output stage magnetic ring.

In some embodiments, the respective driving disc assembly includes a driving disc guide portion and a driving disc. The driving disc guide portion is coupled to a shaft of the respective reducer output stage, the driving disc is in sliding fit with the driving disc guide portion, and the respective output stage magnetic ring is coupled to the driving disc guide portion.

In the instrument drive for the surgical robot of the disclosure, the output stage magnetic ring is directly installed on the output disc guide portion, simplifying the internal structure of the instrument driver.

In some embodiments, the driving disc and the driving disc guide portion are made of a non-magnetic material or a weak magnetic material.

In the instrument drive for the surgical robot of the disclosure, materials of components close to the output stage magnetic ring are all non-magnetic material or a weak magnetic material, avoiding the magnetization of these materials and affecting the measurement accuracy and precision of the encoder, and greatly improving the measurement accuracy and precision.

In some embodiments, the respective output stage magnetic ring is mounted to the respective reducer output stage and close to the output stage bearing of the respective reducer, and the respective output stage encoder is mounted to a side of the output sensing plate facing the output stage bearing of the respective reducer.

In the instrument drive for the surgical robot of the disclosure, the output stage encoder can be located above the output stage magnetic ring.

In some embodiments, the output stage bearing of the respective reducer includes an outer bearing and an inner bearing, and the respective output stage magnetic ring is close to the outer bearing.

In the instrument drive for the surgical robot of the disclosure, double bearing is adopted in a shaft system of the reducer, which can reduce the impact of axial movement amount of the output stage shaft on measurement of the sensor and the sensor measurement error caused by the up-and-down movement of the magnetic ring.

In some embodiments, the outer bearing is spaced apart from the inner bearing, or the outer bearing abuts against the inner bearing.

In the instrument drive for the surgical robot of the disclosure, a distance between double bearings in the shaft system of the reducer can be adjusted according to the actual space inside the instrument drive.

In some embodiments, the outer bearing is made of a non-magnetic material or a weak magnetic material.

In the instrument drive for the surgical robot of the disclosure, materials of components close to the output stage magnetic ring are all non-magnetic materials or weak magnetic materials, which greatly improves the accuracy and precision of measurement by avoiding the magnetization of these materials and thus affecting the encoder's measurement accuracy and precision.

In some embodiments, the plurality of output stage encoders are disposed outside a convex polygon formed by connecting centers of the plurality of reducer output stages.

In the instrument drive for the surgical robot of the disclosure, the effect of respective encoder units interfering with each other can be minimized.

In some embodiments, the respective reducer includes a top cover made of a non-magnetic material or a weak magnetic material.

In some embodiments, the plurality of reducer output stages are made of non-magnetic materials or weak magnetic materials.

In the instrument drive for the surgical robot of the disclosure, materials of components close to the output stage magnetic ring are all non-magnetic materials or weak magnetic materials, which greatly improves the accuracy and precision of measurement by avoiding the magnetization of these materials and thus affecting the encoder's measurement accuracy and precision.

In some embodiments, each of the plurality of motors includes an input bearing and an output bearing.

In the instrument drive for the surgical robot of the disclosure, double bearing is adopted in a motor shaft system, and the axial movement of the motor shaft on the measurement of the sensor can be reduced, such that a sensor measurement error caused by the upward and downward movement of the magnetic ring can be greatly reduced.

In some embodiments, the input bearing is made of a non-magnetic material or a weak magnetic material.

In the instrument drive for the surgical robot of the disclosure, materials of components close to the input stage magnetic ring are all non-magnetic materials or weak magnetic materials, which greatly improves the accuracy and precision of measurement by avoiding the magnetization of these materials and thus affecting the encoder's measurement accuracy and precision.

In some embodiments, the instrument drive further includes: a plurality of input stage magnetic rings. A respective input stage magnetic ring of the plurality of input stage magnetic rings is coaxially arranged with an input stage of the respective motor and is configured to rotate synchronously with the input stage of the respective motor; and a plurality of input stage encoders, fixedly disposed in the instrument drive and a respective input stage encoder of the plurality of input stage encoders being configured to detect rotation of a respective input stage magnetic ring.

In the instrument drive for the surgical robot of the disclosure, a compact rotation detection scheme at the input end of the motor and the output end of the reducer can be achieved. The detection value of the output stage encoding detecting unit can be compared with the detection value of the input stage encoding detecting unit, and then the error that may occur in the output stage can be compensated by software control, so that the control accuracy of the instrument drive is higher.

In some embodiments, the respective input stage magnetic ring is coaxially arranged with a sensing unit of a respective input stage encoder.

In the instrument drive for the surgical robot of the disclosure, the respective input stage magnetic ring being coaxially arranged with a sensing unit of a respective input stage encoder can improve accuracy of detection of the rotation angle at the input end of the motor.

In some embodiments, the respective motor includes a bottom cover, the bottom cover being made of a non-magnetic material or a weak magnetic material; and/or the respective motor includes a base, the base being made of a non-magnetic material or a weak magnetic material; and/or the respective motor includes a housing, the housing being made of a non-magnetic material or a weak magnetic material.

In some embodiments, In the instrument drive for the surgical robot of the disclosure, materials of components close to the input stage magnetic ring are all non-magnetic materials or weak magnetic materials, which greatly improves the accuracy and precision of measurement by avoiding the magnetization of these materials and thus affecting the encoder's measurement accuracy and precision.

According to a second aspect, embodiments of the disclosure provide a surgical robot, which includes the instrument drive for the surgical robot described above.

According to the surgical robot of the present disclosure, the output stage magnetic ring is coaxial with the reducer output stage, and the output stage encoder is disposed in the radial direction of the reducer output stage, so that the detection of the rotational position of the driving disc can be realized at an axial height of less than 4.5mm and within a space of 7.5mm from the central axis of the reducer in the radial direction of the reducer. In this way, the internal structure of the instrument driver can be simplified, and the internal space of the instrument drive can be greatly saved, so that the external dimension of the instrument drive is smaller and the structure of the surgical robot is better. In addition, in the disclosure, errors such as clearance or deformation caused by complex structure can be eliminated, the rotation angle of the output stage can be directly measured, measurement of the rotation angle of the output stage is more accurate, and control accuracy for end instruments is higher.

### BRIEF DESCRIPTION OF THE DRAWINGS

The following drawings of this disclosure are herein used as a part of this disclosure for understanding the disclosure. Embodiments of the present disclosure and their description are shown in the accompanying drawings to explain the principles of the present disclosure.
FIG. 1 is an exploded perspective view of a driving assembly of a surgical robot according to an optional embodiment of the present disclosure.
FIG. 2 is a side sectional view of an instrument drive according to an optional embodiment of the present disclosure.
FIG. 3 is a front sectional view of an instrument drive according to an optional embodiment of the present disclosure.
FIG. 4 is a perspective view of an instrument drive without part of a housing of the instrument drive according to an optional embodiment of the present disclosure.
FIG. 5 is an example of a partial sectional view taken along line A-A in FIG. 4.
FIG. 6 is a perspective view of an instrument drive without part of a housing of the instrument drive according to an optional embodiment of the present disclosure.
FIG. 7 is yet another example of a partial sectional view taken along line A-A in FIG. 4.

### DETAILED DESCRIPTION OF THE EMBODIMENTS

In the following description, numerous specific details are given to provide a more thorough understanding of the disclosure. However, it will be apparent to those skilled in the art that embodiments of the present disclosure may be practiced without one or more of these details. In other examples, some technical features well known in the art are not described in order to avoid confusion with embodiments of the present disclosure.

In order to thoroughly understand the embodiments of the present disclosure, detailed structures will be set forth in the following description. Apparently, the implementation of the embodiments of the present disclosure is not limited to the particular details familiar to those skilled in the art.

In order to achieve accurate, safe, and high-response control for end effectors of surgical instruments, it is necessary to perform sensing detection on a power unit, to determine a position of a rotor of a motor and a position of an output end of a driving device, so as to realize closed-loop control.

At present, when measuring a position of a revolute at the end of the driving device, it is necessary to have a tooth-shaped outer side of an output disc at a last stage of a gearbox, to define a notch on an outer wall of a reducer, and to arrange a linkage shafting system meshing with an external gear and a Hall sensing unit at a position parallel to an axis of a driving disc. The inventor found that there are some problems existed in the above scheme. For example, many parts and complicated installation are required. In addition, for a pair of gears meshing with each other, there may have a transmission backlash that could not be completely eliminated, which may cause an error term to measurement of an accurate position of the output device at the end. The measurement unit is far away from a driving disc unit that needs to be detected, and deformation from the driving disc to an output at a last stage of the reducer is unable to be reflected in the measurement, which may further reduce the measurement accuracy.

To overcome or address at least one of the above problems, embodiments of the present disclosure provide an instrument drive for a surgical robot and the surgical robot including the instrument drive, optionally implemented by one or more of the following embodiments. Optional embodiments of the present disclosure are described below with reference to the accompanying drawings.

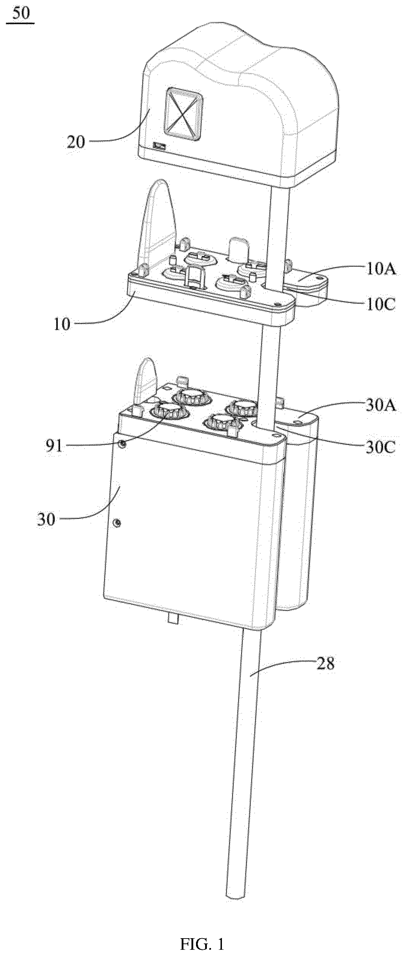

According to an optional embodiment of the present disclosure, a surgical robot is provided. As shown in FIG. 1, the surgical robot includes a driving assembly 50. The driving assembly 50 is mounted to a sliding arm of the surgical robot. The driving assembly 50 includes an instrument drive 30, a sterile adapter 10, and a surgical instrument box 20. The instrument drive 30 is connected to the sliding arm of the surgical robot and is movable on the sliding arm in a controllable manner. The sterile adapter 10 is connected to the instrument drive 30. The surgical instrument box 20 is connected to the sterile adapter 10. Specifically, the sterile adapter 10 is connected to an upper surface 30A of the instrument drive 30 and the surgical instrument box 20 is connected to an upper surface 10A of the sterile adapter 10. The sterile adapter 10 is sandwiched between the instrument drive 30 and the surgical instrument box 20, to operably connect sterilized surgical instruments (e.g., forceps, scissors, clamps, etc.,) with the non-sterilized instrument drive 30. After mounted to the sliding arm, the instrument drive 30, the sterile adapter 10, and the surgical instrument box 20 are all positioned on a same side of the sliding arm, and are movable as a whole relative to the sliding arm.

The surgical instrument is connected to the surgical instrument box 20. The instrument drive 30 is configured to provide, through the sterile adapter 10, a driving force to a transmission mechanism inside the surgical instrument box 20 at the back end of the surgical instrument for achieving pitch, yaw, and grip of an end effector of the surgical instrument. Specifically, the surgeon controls, at a console side, the instrument connected to the drive at a surgical side, to control an internal mechanism of the instrument drive 30. The instrument drive 30 is connected to the sterile adapter 10, and the sterile adapter 10 is connected to the back end of the surgical instrument through the surgical instrument box 20. After connection is completed, a driving disc 91 of the instrument drive 30 may drive a coupling member of the sterile adapter 10, the coupling member of the sterile adapter 10 drives a driven disc of the surgical instrument box 20, the driven disc of the surgical instrument box 20 may drive a steel wire for pulling the surgical instrument, and one end of the steel wire is connected to the end effector of the surgical instrument, thereby achieving pitch, yaw, and grip of the surgical instrument. The steel wire for pulling the surgical instrument is received in an elongated tube 28 of the surgical instrument box 20. The elongated tube 28 is generally disposed at an end of the surgical instrument box 20 farther away from the sliding arm.

Alternatively, the elongated tube 28 extends in a direction parallel to an extending direction of the sliding arm. The sterile adapter 10 defines a notch 10C, running through the sterile adapter 10, for allowing the elongated tube 28 to pass through the sterile adapter 10. The instrument drive 30 defines a notch 30C, running through the instrument drive 30, for allowing the elongated tube 28 to pass through the instrument drive 30.

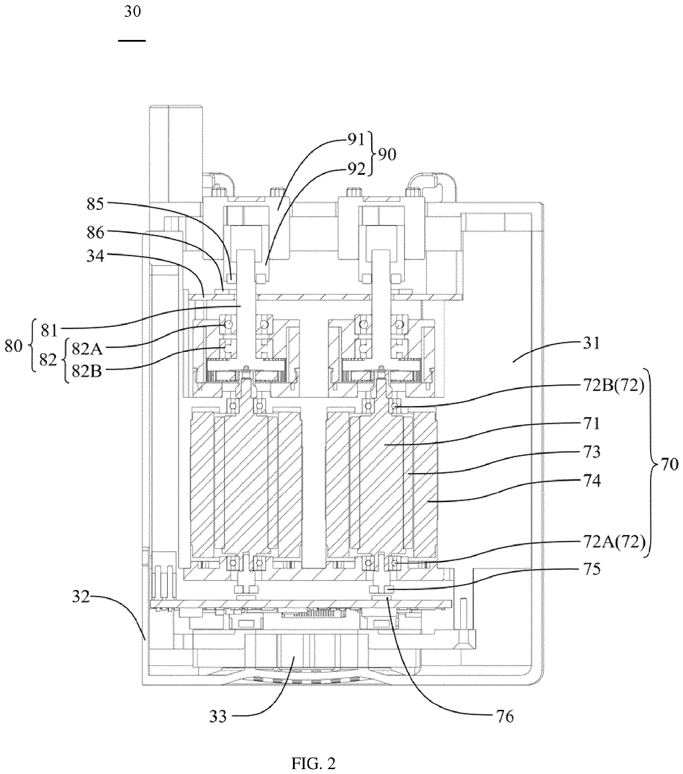

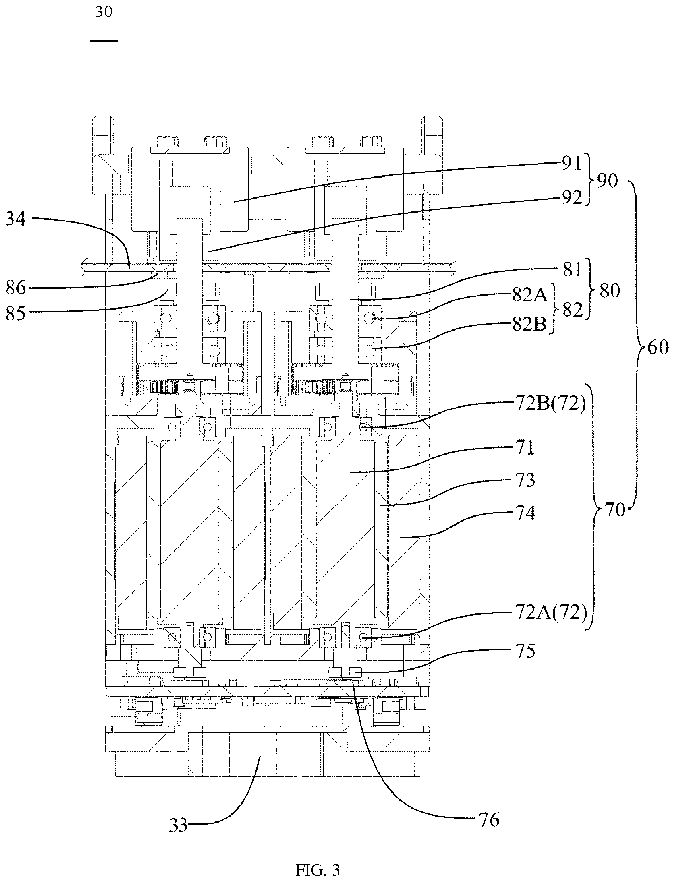

As shown in FIGS. 2 and 3, in order to achieve control of the surgical instrument at the end, the instrument drive 30 generally includes a base 31, a housing 32, a cooling system 33 (e.g., at least one fan and vent), and a plurality of transmission assemblies 60. Each transmission assembly 60 includes a motor 70, a reducer 80, a reducer output stage 81, and a driving disc assembly 90. Each transmission assembly 60 is configured to control movement of the surgical instrument at the end in a direction. In the transmission assembly 60, the motor 70, the reducer 80, the reducer output stage 81, and the driving disc assembly 90 are provided in sets. The motor 70 is configured to drive a center gear of the reducer 80 to rotate, where the center gear is connected to the reducer output stage 81, such that the reducer 80 drives the driving disc assembly 90 (including the driving disc 91) to rotate, and thus the driving disc 91 can drive a driven disc in the surgical instrument box 20. Since the end effector of the surgical instrument needs to achieve pitch, yaw, grip, and other operations, there is a need to provide three to five power sources to provide power. That is, the instrument drive 30 needs to be provided with three to five transmission assemblies 60.

In the instrument drive 30 of the present disclosure, the motor 70 is a power source that is used to provide power, and the reducer 80 is used to adjust torque and reduce a rotational speed. In order to accurately control motors 70, a high-precision sensor unit is provided at an input end of each motor. In addition, in order to accurately monitor a position and a direction of the driving disc 91, a high-precision sensor is further provided at a side close to the driving disc 91.

Specifically, the motor 70 includes a motor shaft 71, a motor bearing 72, a motor stator 73, a motor rotor 74, and the like. In an optional embodiment of the present disclosure, the instrument drive 30 is provided with an input stage encoding detecting unit at an input end of the motor, such as an input stage magnetic ring 75 and an input stage encoder 76, for detecting a rotation angle of the motor 70. The input stage magnetic ring 75 is coaxial with the input stage of the motor 70 and is configured to move synchronously with the input stage (e.g., motor shaft 71) of the motor 70. The input stage encoder 76 is fixedly disposed in the instrument drive 30 and configured to detect rotation of the input stage magnetic ring 75. The input stage magnetic ring 75 is arranged coaxially with a sensing unit of the input stage encoder 76. When the input stage magnetic ring 75 rotates with the motor shaft 71, the input stage encoder 76 senses the rotation of the input stage magnetic ring 75, so as to monitor rotation of the input end of the motor. In order to reduce influence of axial movement of the motor shaft 71 on measurement of the sensor, double bearing is adopted in a motor shaft system, and includes a motor input bearing 72A and a motor output bearing 72B. In this way, the axial movement amount of the motor shaft 71 can be controlled at several wire levels (below 0.1 mm), and a sensor measurement error caused by the upward and downward movement of the magnetic ring may be greatly reduced. In addition, the motor input bearing 72A, a bottom cover of the motor, the base 31, and the housing 32 are made of a non-magnetic material or a weak magnetic material (for example, non-magnetic steel with a magnetic permeability of µ≤1.319×10-6H/m, or stainless steel with a magnetic permeability of µ≤1.339×10-6H/m), to reduce the influence on the measurement precision and accuracy of the input stage encoder 76.

The non-magnetic material or the weak magnetic material may include the non-magnetic steel, and/or the stainless steel, or may also include other materials with a magnetic permeability approximate to the magnetic permeability of the magnetic steel or the stainless steel. Furthermore, in the present disclosure, as described above, the use of the non-magnetic material or the weak magnetic material is to reduce the influence on the measurement precision and accuracy of the input stage encoder 76, so that the non-magnetic material or the weak magnetic material is selected, such that the measurement precision and accuracy of the input stage encoder 76 is not affected, or the influence on the measurement precision and accuracy of the input stage encoder 76 is within an allowable error range.

The driving disc assembly 90 includes the driving disc 91 and a driving disc guide portion 92. The driving disc guide portion 92 is coupled to a shaft of the reducer output stage 81, and the driving disc 91 is in sliding fit with the driving disc guide portion 92. The driving disc 91 can be moved up and down along an axial direction of the motor 70 or the reducer 80, so as to achieve flexible engagement with the sterile adapter 10. To monitor the rotation of the driving disc 91, the instrument drive 30 is further provided with an output stage encoding detecting unit, such as an output stage magnetic ring 85 and an output stage encoder 86, at the reducer output stage 81. The output stage magnetic ring 85 is arranged coaxially with the reducer output stage 81 and configured to rotate synchronously with the reducer output stage 81. The output stage encoder 86 is fixedly arranged in the radial direction of the reducer output stage 81 and configured to detect the rotation of the output stage magnetic ring 85, that is, to detect the rotation of the reducer output stage 81 and the rotation of the driving disc 91.

In the present disclosure, rotation encoding detecting units are provided at both the input stage and the output stage of the instrument drive 30, a detection value of the output stage encoding detecting unit can be compared with a detection value of the input stage encoding detecting unit, and then an error that may occur in the output stage can be compensated by software control, so that the control accuracy of the instrument drive 30 is higher.

Specifically, the instrument drive 30 includes an output sensing plate 34, and the output sensing plate 34 is disposed between an output stage bearing 82 of the reducer 80 and the driving disc assembly 90. The output sensing plate 34 defines a through hole for the reducer output stage 81 (specifically, a shaft of the reducer output stage 81) to axially pass through. The output stage encoder 86 is fixedly mounted to the output sensing plate 34.

As shown in FIG. 2, in one embodiment, the output stage magnetic ring 85 is coupled to the driving disc guide portion 92 and can rotate synchronously with the reducer output stage 81 (or the driving disc guide portion 92). The output stage encoder 86 is adjacent to the output stage magnetic ring 85 and mounted to a side of the output sensing plate 34 facing the driving disc assembly 90. The output stage sensing unit is arranged as close as possible to the driving disc 91 of the terminal output member, thus reducing a measurement error caused by stiffness torsion and parallel axis gear meshing (with backlash) of the whole output connection link. In addition, the output stage magnetic ring 85 is directly mounted to the driving disc guide portion 92, and parts of the measurement unit at the output stage are used interchangeably, which greatly reduces the complexity of production and assembly, improves production efficiency, and reduces product cost.

As shown in FIG. 3, in another embodiment, the output stage magnetic ring 85 is mounted to the reducer output stage 81 and close to the output stage bearing 82 of the reducer 80, and the output stage encoder 86 is mounted to a side of the output sensing plate 34 facing the output stage bearing 82 of the reducer 80. Similar to the input stage of the motor, in order to reduce the influence of the axial movement of the output stage shaft on measurement of the sensor, double bearing is adopted in a shaft system of the reducer 80 and includes an output stage outer bearing 82A and an output stage inner bearing 82B, and a pretightening clearance elimination technology is also adopted in the shaft system of the reducer 80. The output stage outer bearing 82A and the output stage inner bearing 82B are arranged in a longitudinal direction (axial direction) of the reducer output stage 81 and may be spaced apart each other or may abut against each other. With the double bearing design, the axial movement amount of the output stage shaft can be controlled at several wire levels (below 0.1 mm), which greatly reduces the sensor measurement error caused by the up-and-down movement of the magnetic ring. Optionally, the output stage magnetic ring 85 is located close to the output stage outer bearing 82A and disposed between the output stage outer bearing 82A and the output sensing plate 34.

In embodiments of the disclosure, the output stage magnetic ring 85 is mounted to the reducer output stage 81 and coaxial with the reducer output stage 81, and the output stage encoder 86 is disposed in the radial direction of the reducer output stage 81, so that the detection of the rotational position of the driving disc 91 can be realized at an axial height of less than 4.5mm and within a space of 7.5mm from the central axis of the reducer 80 in the radial direction of the reducer 80. In this way, it is possible to make up the shortcomings of a series of complex designs in related technologies, such as arranging gears and a sensor transmission shaft system in a direction parallel to the output axis of the reducer 80.

Optionally, in the instrument drive 30, materials of components such as the driving disc 91, the driving disc guide portion 92, the output stage outer bearing 82A of the reducer 80, the reducer output stage 81, and a top cover (not shown) of the reducer that are close to the output stage magnetic ring 85 are all non-magnetic materials or weak magnetic materials (e.g., non-magnetic steel with a magnetic permeability of µ≤1.319×10-6H/m, or stainless steel with a magnetic permeability of µ≤1.339×10-6H/m), such that magnetization of these materials to affect the measurement precision and accuracy of the output stage encoder 86 can be avoided, and the measurement precision and accuracy can be greatly improved. Similarly, the non-magnetic materials or the weak magnetic materials as defined herein are all materials of which influence on the measurement precision and accuracy of the output stage encoder 86 is within an error allowable range of the technical scheme of the present disclosure, and whether the materials are suitable for the technical scheme of the present disclosure can be determined experimentally by those skilled in the art.

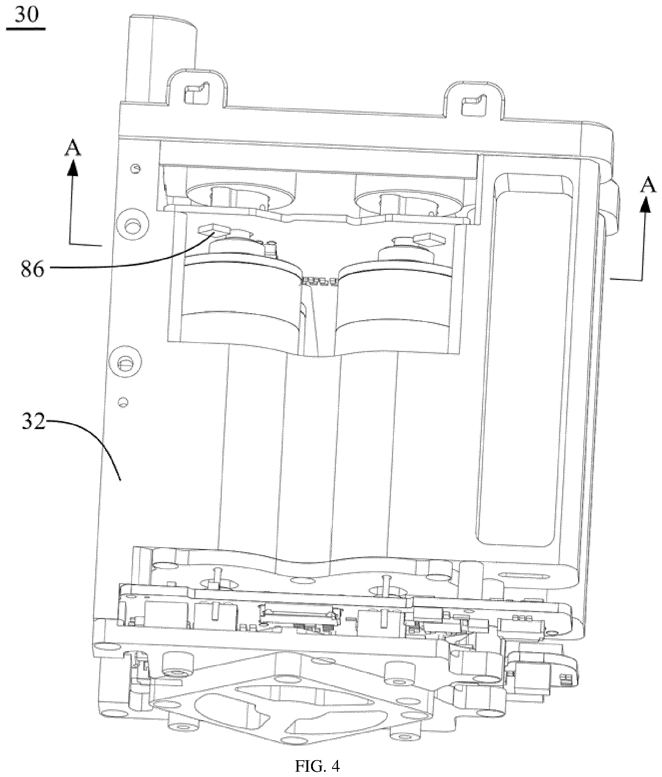

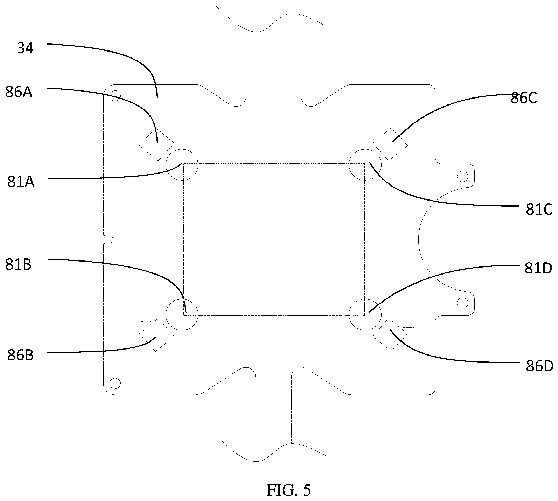

As shown in FIGS. 4 and 5, a respective output stage encoder 86 is disposed in a radial direction of a respective reducer output stage 81. When the instrument drive 30 includes the plurality of transmission assemblies 60, lines connecting centers of a plurality of reducer output stages 81 (e.g., 81A, 81B, 81C, and 81D) form a convex polygon (e.g., quadrilateral), and output stage encoders 86 (e.g., 86A, 86B, 86C, and 86D) are located outside the convex polygon to minimize the influence of interference between the respective encoder units. Moreover, by experimental verification, this above arrangement can reduce the mutual influence of the encoders to a degree that it is unnecessary to arrange a magnetic shielding material near each sensor unit, which may greatly reduce the complexity of installation and debugging.

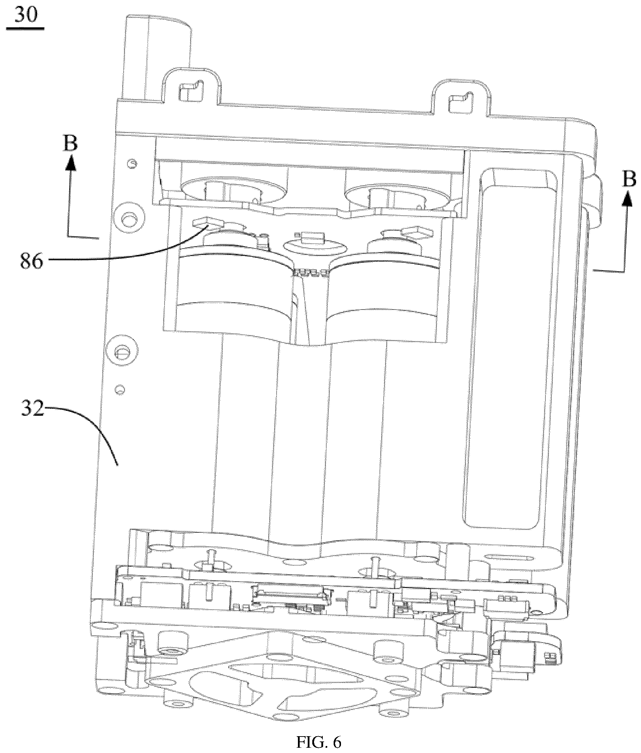

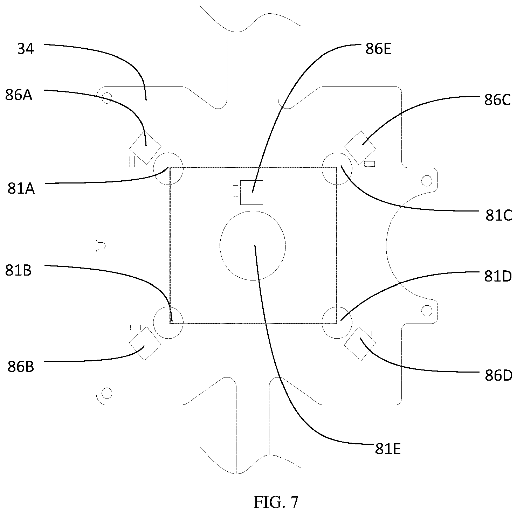

As shown in FIGS. 6 and 7, when the number of transmission assemblies 60 increases, the respective output stage encoder 86 is disposed in the radial direction of the respective reducer output stage 81. The lines connecting the centers of the plurality of reducer output stages 81 (e.g., 81A, 81B, 81C, and 81D) relatively located on the outer side form a convex polygon (e.g., a quadrilateral), and the output stage encoders 86 (e.g., 86A, 86B, 86C, and 86D) are located on the outer side of the convex polygon to minimize the influence of mutual interference of the respective encoder units. The encoder (e.g., 86E) corresponding to the reducer output stage 81 (e.g., 81E) relatively located on the inner side is symmetrically arranged with respect to the outer encoders, so that the encoder on the inner side can be located at a position where the effects of the encoders on the outer side can cancel each other out.

In order to provide as large a workspace as possible for the end surgical tool, it is necessary to make the instrument drive 30 at the end of the surgical robot as compact as possible, e.g., reducing dimensions of the instrument drive 30 in all three directions of height, width, and length. In this disclosure, the sensor layout scheme can realize a compact rotation detection scheme at the input end of the motor and the output end of the reducer.

In the present disclosure, the detection value of the output stage encoding detecting unit can be compared with the detection value of the input stage encoding detecting unit, and then the error that may occur in the output stage can be compensated by software control, so that the control accuracy of the instrument drive 30 is higher.

According to the instrument drive 30 for the surgical robot and the surgical robot including the instrument drive 30 of the present disclosure, the output stage magnetic ring 85 is coaxial with the reducer output stage 81, and the output stage encoder 86 is disposed in the radial direction of the reducer output stage 81, so that the detection of the rotational position of the driving disc 91 can be realized at an axial height of less than 4.5mm and within a space of 7.5mm from the central axis of the reducer 80 in the radial direction of the reducer 80. In this way, a series of complicated designs such as gears and sensor transmission shafts arranged in the direction parallel to the output axis of the reducer in the related technologies can be omitted, and the internal space of the instrument drive 30 can be greatly saved, so that the external dimension of the instrument drive 30 is smaller and the structure of the surgical robot is better. In addition, in the disclosure, errors such as clearance or deformation caused by complex structure can be eliminated, the rotation angle of the output stage can be directly measured, measurement of the rotation angle of the output stage is more accurate, and control accuracy for end instruments is higher.

Unless otherwise defined, technical and scientific terms used herein have the same meanings as are commonly understood by those skilled in the art of the present disclosure. Terms used herein are for specific practical purposes only and are not intended to limit the present disclosure. Terms such as "disposed/disposing" that appear herein may denote that either a part is attached directly to another part or a part is attached to another part through middleware. Features described herein in one embodiment may be applied to another embodiment alone or in combination with other features unless the features are not applicable in the other embodiment or otherwise noted.

The present disclosure has been described by way of the above-described embodiments, but it shall be understood that the above-described embodiments are for the purpose of illustration and illustration only and are not intended to limit the present disclosure to the scope of the described embodiments. Those skilled in the art may appreciate that many more variations and modifications may be made in accordance with the teachings of the present disclosure, all of which fall within the scope of the claims of the present disclosure.

## Claims

1. An instrument drive for a surgical robot, the instrument drive comprising a plurality of motors, a plurality of reducers, a plurality of reducer output stages, and a plurality of driving disc assemblies, wherein each respective motor of the plurality of motors is configured to drive a respective reducer to rotate a respective driving disc assembly via a respective reducer output stage, wherein the instrument drive further comprises:
a plurality of output stage magnetic rings, wherein a respective output stage magnetic ring of the plurality of output stage magnetic rings is coaxially disposed with the respective reducer output stage and configured to rotate synchronously with the respective reducer output stage; and
a plurality of output stage encoders, wherein a respective output stage encoder of the plurality of output stage encoders is fixedly disposed in a radial direction of the respective reducer output stage and configured to detect rotation of the respective output stage magnetic ring.

2. The instrument drive of claim 1, wherein the instrument drive further comprises an output sensing plate disposed between an output stage bearing of the respective reducer and the respective driving disc assembly, and the output sensing plate defines a plurality of through holes, wherein a respective through hole of the plurality of through holes allows the respective reducer output stage to pass through, wherein the plurality of output stage encoders are mounted to the output sensing plate.

3. The instrument drive of claim 2, wherein the respective output stage magnetic ring is mounted to the respective driving disc assembly and the respective output stage encoder is mounted to a side of the output sensing plate facing the respective driving disc assembly.

4. The instrument drive of claim 3, wherein the respective driving disc assembly includes a driving disc guide portion and a driving disc, wherein the driving disc guide portion is coupled to a shaft of the respective reducer output stage, the driving disc is in sliding fit with the driving disc guide portion, and the respective output stage magnetic ring is coupled to the driving disc guide portion.

5. The instrument drive of claim 4, wherein the driving disc and the driving disc guide portion are made of a non-magnetic material or a weak magnetic material.

6. The instrument drive of any of claims 2 to 4, wherein the respective output stage magnetic ring is mounted to the respective reducer output stage and close to the output stage bearing of the respective reducer, and the respective output stage encoder is mounted to a side of the output sensing plate facing the output stage bearing of the respective reducer.

7. The instrument drive of claim 6, wherein the output stage bearing of the respective reducer includes an outer bearing and an inner bearing, and the respective output stage magnetic ring is close to the outer bearing.

8. The instrument drive of claim 7, wherein the outer bearing is spaced apart from the inner bearing, or the outer bearing abuts against the inner bearing.

9. The instrument drive of claim 7, wherein the outer bearing is made of a non-magnetic material or a weak magnetic material.

10. The instrument drive of any of claims 1 to 9, wherein the plurality of output stage encoders are disposed outside a convex polygon formed by connecting centers of the plurality of reducer output stages.

11. The instrument drive of any of claims 1 to 10, wherein the respective reducer includes a top cover made of a non-magnetic material or a weak magnetic material.

12. The instrument drive of any of claims 1 to 11, wherein the plurality of reducer output stages are made of non-magnetic materials or weak magnetic materials.

13. The instrument drive of any of claims 1 to 12, wherein each of the plurality of motors includes an input bearing and an output bearing.

14. The instrument drive of claim 13, wherein the input bearing is made of a non-magnetic material or a weak magnetic material.

15. The instrument drive of any of claims 1 to 14, wherein the instrument drive further comprises:
a plurality of input stage magnetic rings, wherein a respective input stage magnetic ring of the plurality of input stage magnetic rings is coaxially arranged with an input stage of the respective motor and is configured to rotate synchronously with the input stage of the respective motor; and
a plurality of input stage encoders, fixedly disposed in the instrument drive, and a respective input stage encoder of the plurality of input stage encoders being configured to detect rotation of a respective input stage magnetic ring.

16. The instrument drive of claim 15, wherein the respective input stage magnetic ring is coaxially arranged with a sensing unit of a respective input stage encoder.

17. The instrument drive of any of claims 1 to 16, wherein
the respective motor includes a bottom cover, the bottom cover being made of a non-magnetic material or a weak magnetic material; and/or
the respective motor includes a base, the base being made of a non-magnetic material or a weak magnetic material; and/or
the respective motor includes a housing, the housing being made of a non-magnetic material or a weak magnetic material.

18. A surgical robot, comprising the instrument drive for the surgical robot of any of claims 1 to 17.
